(19) Europäisches Patentamt
European Patent Office
Office européen des brevets

(11) Veröffentlichungsnummer: **0 000 140**
**B1**

(12) # EUROPÄISCHE PATENTSCHRIFT

(21) Anmeldenummer: 78100145.8

(22) Anmeldetag: 13.06.78

(51) Int. Cl.³: C 07 F 9/54, C 07 C 175/00

(54) **Verfahren zur Herstellung von wässrigen Lösungen bzw. feinteiligen wässrigen Dispersionen von Polyenyltriarylphosphoniumsalzen**

(30) Priorität: 18.06.77 DE 2727384
02.07.77 DE 2729974

(43) Veröffentlichungstag der Anmeldung:
10.01.79 Patentblatt 79/01

(45) Bekanntmachung des Hinweises auf die Patenterteilung:
23.07.80 Patentblatt 80/15

(84) Benannte Vertragsstaaten:
BE CH DE FR GB NL

(56) Entgegenhaltungen:
GB 1 059 673

(73) Patentinhaber: BASF Aktiengesellschaft
Carl-Bosch-Strasse 38
D-6700 Ludwigshafen (DE)

(72) Erfinder: Schulz, Bernhard, Dr.
Kurpfalzring 38
D-6830 Schwetzingen (DE)
Grafen, Paul, Dr.
Suedtiroler Ring 18-20
D-6719 Weisenheim (DE)
Scholz, Hans-Ulrich, Dr.
Zeiss-Strasse 2
D-6840 Lampertheim (DE)
Grassner, Hans, Dr.
Wielandtstrasse 4a
D-6900 Heidelberg (DE)
Reif, Werner, Dr.
Pierstrasse 5
D-6710 Frankenthal (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 000 140 B1

Verfahren zur Herstellung von wäßrigen Lösungen bzw. feinteiligen wäßrigen Dispersionen von Polyenyltriarylphosphoniumsalzen

Die vorliegende Erfindung betrifft ein Verfahren zur Herstellung von wäßrigen Lösungen bzw. wäßrigen feinteiligen Dispersionen von Polyenyltriarylphosphoniumsalzen der allgemeinen Formel I

$$\left[R=P(Ar)_3\right]^{\oplus} X^{\ominus} \qquad I,$$

in der R für einen aliphatischen, cycloaliphatisch-aliphatischen oder aromatisch-aliphatischen Polyenylrest mit 5 bis 20 C-Atomen steht, X das Anionäquivalent einer starken Säure bezeichnet und Ar einen Arylrest, insbesondere den Phenylrest bedeutet.

Einige der Verbindungen I (z.B. I mit R = ß-Jonylidenäthyl) eignen sich unmittelbar als Schädlingsbekämpgungsmittel, z.B. zur Bekämpfung von Wasserschnecken, andere dienen als Zwischenprodukte für organische Synthesen, insbesondere auf dem Carotinoidgebiet (vgl. u.a. die DT-PS 1 203 264 und 1 046 046). Sowohl für die unmittelbare Verwendung als auch für die Verwendung für weitere Synthesen, wie z.B. für die Herstellung von synmetrischen Carotinoiden gemäß der DT—OS 2 505 869, empfehlen sich häufig wäßge Lösungen bzw. wäßrige, feinteilige Dispersionen der Verbindungen I.

Nach Houben-Weyl, "Methoden der Organischen Chemie", Band XII/1, Seiten 90 ff, erhält man Phosphoniumsalze aus Triphenylphosphin, einer Säure und einem Alkohol gemäß der Reaktionsgleichung

$$(C_6H_5)_3P + HX + HOR \longrightarrow \left[(C_6H_5)_3\overset{+}{P}-R\right] X^- + H_2O$$

Als Lösungsmittel für diese Umsetzung sind niedere aliphatische Alkohole, niedere Carbonsäuren, wie HCOOH oder $CH_3COOH$, Aceton, oder die üblichen wasserunlöslichen Lösungsmittel wie Benzol, Toluol, Tetrahydrofuran, Acetonitril, Methylenchlorid, Chloroform, Diäthyläther, Dioxan und Ester, wie Methyl- und Diäthylacetat bekannt, d.h. es fallen bei den üblichen Herstellungsmethoden Lösungen von I in den genannten Lösungsmitteln an. Eine direkte Umsetzung von Triphenylphosphin mit einer Säure end einem Polyenalkohol in Wasser zwecks Herstellung einer wäßrigen Lösung von Polyenyltriphenylphosphoniumsalzen (I) erscheint aussichtslos, da einerseits weder das Triphenylphosphin noch der Polyenalkohol in Wasser löslich sind, andererseits mit einer großen Hydrolyseempfindlichkeit von I bei höheren Temperaturen zu rechnen ist und man annehmen kann, daß das Vorhandensein größerer Mengen eines der bei der gewünschten Reaktion sich bildenden Produkte, das Reaktionsgleichgewicht ungünstig beeinflußt.

Nach dem bekannten Verfahren erhält man die besten Ausbeuten an I bei Verwendung von niederen Alkoholen, wie Äthanol, Isopropanol, Isobutanol, n-Propanol, n-Butanol und insbesondere Methanol als Lösungsmittel. So werden als Ausgangslösungen für die Herstellung wäßriger I-Lösungen bevorzugt Lösungen von I in den genannten niederen Alkoholen, insbesondere methanolische I-Lösungen eingesetzt werden. Aber auch die Überführung von Lösungen von I in einem der anderen oben genannten Lösungsmittel in wäßrige i-Lösungen ist von Interesse. Die Herstellung möglichst lösungsmittelfreier, wäßriger I-Lösungen aus den entsprechenden Lösungen in organischen Lösungsmitteln durch restloses destillatives Entfernen des Lösungsmittels und Aufnahme von I in Wasser ist wegen hierbei unvermeidlichen örtlichen Überhitzungen und der großen thermischen Empfindlichkeit der Verbindungen I (s.l.c. S. 105) mit großen Ausbeuteverlusten verbunden und daher techisch kaum realisierbar. Versetzt man die Lösungen von I in organischen Lösungsmitteln zuerst mit Wasser und versucht das Lösungsmittel danach abzudestillieren, so beginnen die Lösungen im allgemeinen so heftig zu schäumen, daß eine reguläre Destillation nicht mehr möglich ist. Auch die Verwendung von Lösungsmitteln, in denen I weniger gut löslich ist, anschließendes Auskristallisierenlassen von I aus dem Solvens und Aufnahme von I in Wasser erschient wegen des hohen Aufwandes an Zeit und Apparaten sowie durch die bei Kristallisationsverfahren unvermeidlichen Ausbeuteverluste nicht sehr attraktiv Andererseits kann man die Umsetzung auch nicht ohne Lösungsmittel ausführen, da sonst (insbesondere bei Verwendung von Schwefelsäure als Protonendonator) Zersetzungen des Alkohols oder Oxidation des Phosphins zum entsprechenden Phosphinoxid auftreten.

Auch die GB-PS 1 059 673, die ein Verfahren zur Herstellung von Polyen-Verbindungen betrifft, ergab keinen Hinweis über einen vorteilhaften Weg zur Herstellung von wäßrigen lösungen bzw. feinteiligen wäßrigen Dispersionen von Polyentriarylphosphoniumsalzen.

Es war daher die Aufgabe der Erfindung, auf möglichst einfache und wirtschaftliche Weise wäßrige Lösungen bzw. feinteilige, wäßrige Dispersionen von I herzustellen.

Es wurde nun überraschenderweise ein sehr vorteilhaftes Verfahren zur Überführung etwa 10- bis 70-gewichtsprozentiger Lösungen von Polyenyltriarylphosphoniumsalzen der allgemeinen Formel I

$$[R-P(Ar)_3]^+ X^- \qquad (I),$$

in der R für einen aliphatischen, cycloaliphatisch-aliphatischen oder aromatisch-aliphatischen Polyenylrest mit 5 bis 20 C-Atomen steht, X das Anionäquivalent einer starken Säure bezeichnet und Ar einen Arylrest, insbesondere den Phenylrest bedeutet, in einem organischen Lösungsmittel in 10- bis 70-gewichtsprozentige, weitgehend lösungsmittelfreie, wäßrige Lösungen bzw. feinteilige Dispersionen, gefunden, das dadurch gekennzeichnet ist, daß man das Lösungsmittel sowie sonstige von der Synthese von I herstammenden, wasserdampfflüchtigen Verbindungen aus der auf 30 bis 120°C gehaltenen Lösung mit Wasserdampf abtreibt, wobei man einen Teil des Wasserdampfes zur Erzeugung der wäßrigen Lösungen kondensieren läßt. Beim Abkühlen unter Rühren bilden sich aus diesen homogenen, viskosen I-Lösungen feinteilige Dispersionen.

Es wurde weiterhin überraschend gefunden, daß dieses Verfahren besonders gut kontinuierlich durchzuführen ist, indem man die organische Lösung von I von oben kontinuierlich in eine Kolonne, vorzugsweise in eine Füllkörperkolonne leitet, sie im Gegenstrom mit dem Wasserdampf in Kontakt bringt, hierbei einen Teil des Wasserdampfes kondensieren läßt und die gebildete, wäßrige Lösung von I kontinuierlich aus dem unteren Teil der Kolonne abzieht. Die Dosierung der I-Lösung sowie des Wasserdampfes kann unschwer so eigerichtet werden, daß man die gewünschte wäßrige I-Lösung in Form einer homogenen, viskosen Lösung unmittelbar als Sumpfprodukt abziehen kann.

Das heiße Sumpfprodukt wird zweckmäßig kontinuierlich in einen Rührbehälter überführt. Durch Kühlen und Rühren erhält man im allgemeinen aus dieser heißen, wäßrigen I-Lösung eine Dispersion, die feinteiliges, kristallines Phosphoniumsalz, dispergiert in Wasser bzw. in wäßriger Phosphoniumsalzlösung, enthält.

Es ist bekannt, daß die Schmelzpunkte bzw. die Zersetzungspunkte der Triarylphosphoniumsalze, insbesondere der Triphenylphosphoniumhydrogensulfate über 100°C liegen (ß-Jonylidentriphenylphosphoniumhydrogensulfat schmilzt beispielsweise bei 183-85°C unter Zersetzung, Axerophthyltriphenylphosphoniumhydrogensulfat bei 188-190°C unter Zersetzung) und daß die Löslichkeit der Triarylphosphoniumsalze in Wasser vor allem bei Raumtemperatur gering ist. Daher war zu erwarten, daß bei einer kontinuierlichen Fahrweise in einer Kolonne mit abnehmender Konzentration des organischen Lösungsmittels in Abtriebsteil der Kolonne die Phosphoniumsalze teilweise auskristallisieren und die Kolonne verstopfen. Überraschenderweise treten bei dem erfindungsgemäßen Verfahren solche Schwierigkeiten jedoch nicht auf.

Das erfindungsgemäße Verfahren kann sowohl diskontinuierlich als auch kontinuierlich durchgeführt werden. Überraschenderweise ist weder bei diskontinuierlicher noch bei kontinuierlicher Verfahrensvariante eine störende Schaumbildung zu beobachten.

Zur Durchführung der diskontinuierlichen Variante hält man die Lösung von I in dem organischen Lösungsmittel in einem Reaktionsgefäß auf einer Temperatur kurz unterhalb der Siedetemperatur des Lösungsmittels, leitet hierein Wasserdampf ein und läßt das hierdurch verdampfende Lösungsmittel, sonstige von der Synthese von I herstammende wasserdampfflüchtige Verbindungen sowie einen Teil des Wasserdampfs aus dem Reaktionsgefäß abdestillieren.

Zu den Betriebsbedingungen für die kontinuierliche erfindungsgemäße Variante läßt sich allgemein gültig etwa folgendes sagen: Die Temperatur der Eingangslösung sollte unterhalb der Siedetemperatur des Lösungsmittels liegen; die Sumpftemperatur soll etwa 100°C betragen; der Zulauf ist so zu regulieren, daß das Lösungsmittel auf dem Wege der Lösung durch die Kolonne an den Füllkörpern weitgehend verdampft. Für geringen Zulauf genügt eine geringe Kolonnenhöhe, für größere Durchsätze muß die Kolonnenkapazitat entsprechend großen sein. Im einzelnen sind die geeigneten Betreibsbedingungen anhand einiger Vorversuche unschwer zu ermitteln, so daß sich eingehende Ausführungen hierfür erübrigen. Dies gilt auch für des Arbeiten bei niedrigerem oder höherem Druck als Normaldruck.

Der gute Erfolg des erfindungsgemäßen Verfahrens ist von der Art der Polyenreste in den Phosphoniumsalzen I nach bisherigen Beobachtungen praktisch nicht abhängig. Da die Wittigsche Ylid-Synthese vornehmlich zur Herstellung von Verbindungen der Carotinoidreihe, vor allem des Vitamin A und dessen Derivaten sowie des $\beta$-Carotins selbst dient, haben solche Polyenylreste die größte Bedeutung, welche Bausteine für diese Carotinoide sind. Genannt seien vor allem solche Phosphoniumsalze, in denen R für den $\alpha$-oder $\beta$-Jonylidenäthylrest (IIa bzw. IIb),

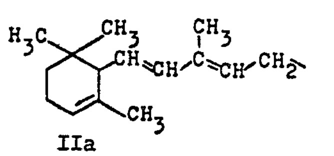
IIa

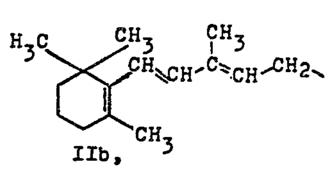
IIb,

deren Methylhomologen oder den Axerophtylrest (III)

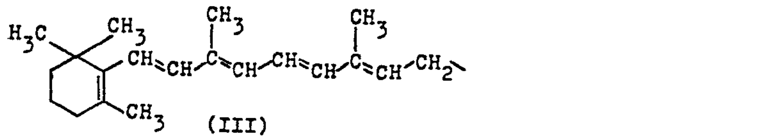

(III)

und dessen Methylhomologen. Allgemein kommen als Polyenylreste solche mit 5 bis 20 C-Atomen und mindestens 2 konjugierten Doppelbindungen in Betracht, wobei eine der konjugierten Dopplebindungen auch eine Kohlenstoff-Sauerstoffbindung sein kann, wie z.B. in dem Rest O=C—C=C—C—. Diese Reste können auch weitere —C=C-Gruppierungen enthalten und u.a. Hydroxyl-, Methoxy- oder Acetoxygruppen als Substituenten tragen. Der cycloaliphatische Rest kann auch durch einen aromatischen Rest, wie Phenyl oder alkyliertes, insbesondere methyliertes Phenyl, ersetzt werden.

Auch die Art des Anions im Polyenyltriarylphosphoniumsalz ist für den Erfolg des erfindungsgemäßen Verfahrens ohne Belang. Im allgemeinen werden die Phosphoniumsalze starker Säuren wie $N_2SO_4$, HCl, HBr, HCOOH und $H_3PO_4$ verwendent, so daß X in I für $HSO_4^{\ominus}$, $Cl^-$, $Br^-$, $HCOO^{\ominus}$ oder $H_2PO_4^{\ominus}$, vorzugsweise für $HSO_4^{\ominus}$ steht.

Das erfindungsgemäße Verfahren ist im Prinzip geeignet für die Überführung von I-Lösungen in allen organischen Lösungsmitteln, die beim Einblasen von Wasserdampf einen Beladungsanteil des Wasserdampfes mit organischem Lösungsmittel von 10% und mehr betragen und die einen Siedepunkt von etwa 50 bis 150°C haben. Besondere Bedeutung hat das Verfahren für diejenigen Lösungsmittel, in denen die Herstellung der Polyenyltriarylphosphoniumsalze mit besonders guten Ausbeuten gelingt und in denen die Phosphoniumsalze gut löslich sind. Genannt seien niedere Akohole, wie Methanol, Äthanol, n-Propanol, iso-Propanol, n-Butanol und iso-Butanol; niedere Carbonsäuren, wie HCOOH und Essigsäure sowie Aceton und Methylenchlorid, Mit besonderem Vorteil verwendet man das erfindungsgemäße Verfahren für die Überprüfung methanolischer Lösungen von Polyenyltriarylphosphoniumsalzen.

Die Herstellung der Lösungen von I in organischen Lösungsmitteln erfolgt nach üblichen Methoden aus dem Triarylphosphin, insbesondere Triphenylphosphin, einer starken Säure, wie HCl, HBr, HCOOH, $H_3PO_4$ oder insbesondere Schwefelsäure und einer Verbindung R-X, wobei X vorzugswiese eine freie Hydroxylgruppe oder eine mit einer niederen Carbonsäure, wie Essigsäure veresterte Hydroxylgruppe ist, Auch andere Methoden zur Herstellung von Triarylphosphoniumsalzen können selbstverständlich benutzt werden.

Geringe Mengen Triphenylphosphin oder der Ausgansvervindung R-X, sowie von Nebenprodukten, die sich bei dieser Quaternierungsreaktion bilden, sind teilweise mit Wasserdampf flüchtig, so daß das erfindungsgemäße Verfahren den Vorteil der Herstellung von reinen wäßrigen I-Lösungen bzw. reinen feinteiligen wäßrigen Dispersionen bietet. Da die Herstellung von Lösungen von I in organischen Lösungsmitteln nicht Gegenstand der Erfindung ist, erübrigen sich nähere Angaben hierüber.

Menge und Temperatur des erforderlichen Wasserdampfs richten sich nach den Gegebenheiten des Einzelfalls. Handelt es sich z.B. um reine methanolische I-Lösungen, so sind zur Entfernung von 1 kg Methanol etwa 1 - 3 kg Wasserdampf von 100°C erforderlich. Dieser Wert ermäßigt sich mit steigender Temperatur und wird größer bei fallender Temperatur und bei vermindertem Druck, so daß man bei Anwendung höherer Dampftemperaturen konzentrierte wäßrige I-Lösungen erhält. Enthalten die methanolischen I-Lösungen noch Verunreinigungen (in der Regel sind es etwa 1 bis 10 Molprozent von I), so ist zu deren Austreibung zusätzlich Wasserdampf erforderlich. Aufgrund dieser Angaben ist es dem Fachmann möglich, die optimalen Verfahrensbedingungen, darunter die Einstellung des Rücklaufverhältnisses, mittels einiger Vorversuche zu ermitteln. Durch geeignete Auslegung der Kolonne gelingt es z.B. ein 80-90 prozentiges Methanol über den Kopf der Kolonne abzudestillieren.

Bei der Überprüfung von Lösungen von Polyenyltriarylphosphoniumhydrogensulfat in einem Lösungsmittel, in dem das Phosphoniumsalz nur schlecht löslich ist, wie in iso-Propanol oder Aceton, empfiehlt es sich, wenn die Lösung von I kontinuierlich ins Reaktionsgefäß gefördert werden soll, der Lösung vor der Dampfbehandlung etwas Wasser zuzusetzen, um auskristallisiertes Phosphoniumsalz in Lösung zu bringen. Generell läßt sich sagen, daß das Vorhandensein von gewissen Mengen (bis etwa 50 Gewichtsprozent) Wasser in der organischen I-Lösung vor und während der Dampfbehandlung sich nicht nachteilig, in einigen Fällen sogar vorteilhaft auswirkt.

Macht man von der kontinuierlichen Fahrweise Gebrauch, so verwendent man als Kolonnen zweckmäßigerweise Füllkörperkolonnen mit einer geeigneten Anzahl theoretischer Böden, um das Lösungsmittel quantitativ abzutrennen. Apparative oder materialmäßige Besonderheiten sind hierbei nur insofern zu beachten als die sauren wäßrigen Phosphoniumsalzlösungen auf das Material der Reaktionsgefäße korrodierend wirken.

In allen Fällen ist es möglich, das Lösungsmittel gänzlich zu entfernen, jedoch reicht es im allgemeinen aus, wenn man auf einen Restlösungsmittelgehalt von 1 bis 2 Gewichtsprozent hinzuarbeitet. Die anfallenden Lösungsmittel-Wassergemische können auf übliche Weise destillativ in ihre Komponenten zerlegt und das Lösungsmittel wieder eingesetzt werden.

Die als Verfahrensprodukte anfallenden wäßrigen I-Lösungen bzw. feinteiligen Dispersionen von I in Wasser, können für weitere Umsetzungen, z.B. für die Herstellung von symmetrischen Carotinoiden,

wie dem β-Carotin oder zur herstellung von Vitamin A verwendet oder nach den üblichen Methoden, z.B. als Schädlingsbekämpfungsmittel, eingesetzt werden.

Beispiel 1

a) Herstellung einer methanolischen Lösung von β-Jonyliden-äthyltriphenylphosphonium-hydrogensulfat

Zu einer Mischung aus 700 ml Methanol und 258 g Triphenylphosphin wurden unter Rühren und Kühlen auf 25-30°C nacheinander 99 g Schwefelsäure und 220 g Vinyl-β-jonol (Reinheitsgrad 93%) zugetropft. Nach 12 Stunden wurde 3 x mit je 250 ml Heptan extrahiert. Man erhält 1150 g einer etwa 43%igen methanolischen Lösung von β-Jonyliden-äthyl-triphenylphosphonium-hydrogensulfat.

b) Überführung der methanolischen in eine wäßrige Lösung von β-Jonyliden-äthyl-triphenylphosphonium-hydrogensulfat

Die gemäß 1a erhaltene methanolische Phosphoniumsalzlösung wurde in 90 Minuten von oben auf eine Füllkörperkolonne aus Glas von 50 cm Länge und 3 cm Durchmesser gegeben. Die Kolonne war mit Raschigringen aus Glas gefüllt und isoliert. Am unteren Kolonnenende wurde in den 90 Minuten 3 900 g Wasserdampf eingeblasen. Die wäßrige Phosphoniumsalzlösung bzw. -suspension wurde über einen Siphon aus dem Sumpf der Kolonne ablaufen lassen und in einem Rührkolben gesammelt. Man erheilt ca. 1 400 g einer gut rührbaren, wäßrigen Suspension, die 497 g β-Jonyliden-äthyltriphenylphosphonium-hydrogensulfat enthielt. Dies entspricht einer Ausbeute von 95 % der Theorie.

Beispiel 2

a) Herstellung einer methanolischen Lösung von Axerophthyl-triphenylphosphoniumhydrogensulfat

Zu einer Mischung von 400 ml Methanol und 131 g Triphenylphosphin wurden in 30 Minuten unter Rühren und Kühlen auf 10°C nacheinander 49 g Schwefelsäure und 164 g Vitamin-A-acetat zugegeben. Anschließend wurde das Reaktionsgemisch noch 12 Stunden bei 25°C nachgerührt. Man erhielt 665 g einer etwa 38%igen methanolischen Lösung von Axerophthyltriphenylphosphoniumhydrogensulfat.

b) Überführung der methanolischen in eine wäßrige Phosphoniumsalzlösung

Die gemäß 2a erhaltene methanolsiche Lösung wurde in 60 Minuten von oben auf die in Beispiel 1 beschriebene Füllkörperkolonne gegeben. Am unteren Ende der Kolonne wurden in den 60 Minuten 2 200 g Wasserdampf eingeblasen. Man erheilt etwa 1 000 g Sumpfablauf.

c) Weiterverarbeitung der wäßrigen Phosphoniumsalzlösung

Die erhaltene wäßrige Lösung von Axerophthyltriphenylphosphoniumhydrogensulfat wurde gemäß Beispiel 6 der DOS 25 05 869 zu β-Carotin umgesetzt. Nach Isomerisieren des erhaltenen Produkts in Heptan erhielt man alltrans-β-Carotin in 70%iger Ausbeute, bezogen auf eingesetztes Vitamin-A-acetat.

Beispiel 3

a) 700 ml Eisessig wurden unter Rühren mit 262 g Triphenylphosphin und 90 g Schwefelsäure versetzt. Anschließend wurden zu dem Reaktionsgemisch innerhalb von 2 Stunden unter Rühren und Kühlen 220 g Vinyl-β-jonol (Reinheitsgrad 93%ig) zugetropft. Hierbei wurde darauf geachtet, daß die Temperatur 35°C nicht überstieg. Schließlich wurde das Reaktionsgemisch noch 12 Stunden unter Rühren ausreagieren lassen.

b) Die gemäß 3a erhaltene Lösung von β-Jonyliden-äthyl-triphenylphosphoniumhydrogensulfat in Essigsäure wurde in einen mit Destillationsbrücke und Vorlage versehenen Kolben überführt und in diesen in ca. 2 Stunden bei 30 - 40 mbar 1,67 kg Wasserdampf eingeblasen, wobei die Temperatur auf 40 - 45°C stieg. Man erhielt 1.9 kg Destillat, das im wesentlichen Essigsäure und Wasser enthält und 1,1 kg Sumpf, der 501 g β-Jonylidenäthyltriphenylphosphoniumhydrogensulfat als wäßrige Lösung bzw. Suspension enthält. Das entspricht einer Ausbeute von 96%, bezogen auf eingesetztes Vinyl-β-jonol.

Beispiel 4

a) 700 ml Methanol wurden mit 258 g Triphenylphosphin und 100 g kristalliner Phosphorsäure (gelöst in möglichst wenig Wasser) versetzt. Zu dieser Mischung wurden in 2 Stunden unter Rühren 220 g Vinyl-β-jonol (Reinheitsgrad 93%, entsprechend 0,93 Mol) zugetropft. Anschließend wurde noch eine Stunde unter Rückfluß zum Sieden erhitzt. Nach dem Abkühlen wurde noch 3 x mit je 250 ml Heptan extrahiert. Man erhielt 1 150 g einer etwa 34 %igen methanolischen Lösung von β-Jonyliden-äthyl-triphenyl-phosphoniumphosphat.

b) Die gemäß 4a erhaltene methanolsiche Lösung wurde analog Beispeil 1 in der dort beschriebenen Kolonne mit Wasserdampf behandelt. Man erhielt 1 400 g einer 26%igen Suspension von β-Jonyliden-äthyl-triphenylphosphoniumphosphat in Wasser. Das entspricht einer Ausbeute von 70 %, bezogen auf eingesetztes Vinyl-β-jonol.

## Beispiel 5

a) 854 g Ameisensäure wurden unter Rühren mit 262 g Triphenylphosphin versetzt. Zu dieser Mischung wurden innerhalb von 2 Stunden 220 g Vinyl-β-jonol (Reinheitsgrad 83%) zugetropft. Hierbei wurde dafür gesorgt, daß die Temperatur des Reaktionsgemisches 35°C nicht überstieg. Anschließend wurde das Reaktionsgemisch noch 12 Stunden unter Rühren ausreagieren lassen. Man erhielt 1 340 g einer etwa 27 gewichtsprozentigen Lösung von β-Jonylidenäthyl-triphenylphosphoniumformiat.

b) In die gemäß 5a erhaltene Lösung wurde analog Beispeil 3b in der dort beschriebene Apparatur in 6 Stunden bei 40 mbar 1,7 kg Wasserdampf eingeblasen. Man erheilt etwa 1,5 kg Destillat, das Ameisensäure und Wasser enthält, sowie 1,5 kg Sumpf, der 0,65 Mol β-Jonylidenäthyl-triphenylphosphoniumformiat gelöst bzw. suspendiert in Wasser enthält. Dies entspricht einer Ausbeute von etwa 70% der Theorie, bezogen auf eingesetztes Vinyl-β-jonol.

c) Behandelt man die gemäß 5a erhaltene Ameisensäurelösung analog Beispiel 1b kontinuierlich mit Wasserdampf, so erhält man nahezu gleiche Ausbeuten an wäßrigem β-Jonylidenäthyl-triphenylphosphoniumformiat wie gemäß 5b.

## Beispiel 6

a) 550 g Äthanol wurden mit 258,5 g Triphenylphosphin versetzt und zu dieser Mischung zunächst innerhalb von 15 Minuten 99,5 g konzentrierte Schwefelsäure und dann in 60 Minuten 220 g Vinyl-β-Jonol (Reinheitsgrad 95 %) zugetropft. Anschließend wurde das Reaktionsgemisch 20 Stunden bei Raumtemperatur nachreagieren lassen. Man erhielt 1 128 g einer etwa 40-gewichtsprozentigen äthanolischen Lösung von β-Jonyliden-äthyl-triphenylphosphoniumhydrogensulfat.

b) Die gemäß 6a erhaltene Lösung wurde analog Beispiel 1 in der dort beschriebenen Kolonne mit Wasserdampf behandelt. Man erhielt 1 400 g einer wäßrigen Suspension, die 454 g β-Jonylidenäthyl-triphenylphosphoniumhydrogensulfat enthielt. Dies entspricht einer Ausbeute von 85 % bezogen auf eingesetztes Vinyl-β-Jonol.

## Beispiel 7

a) 700 ml Isopropanol wurden mit 258,8 g Triphenylphosphin versetzt und zu dieser Mischung zunächst in 15 Minuten 99,5 g konzentrierte Schwefelsäure und dann in 60 Minuten 220 g Vinyl-β-jonol (Reinheitsgrad 93 %) zugetropft. Das gebildete β-Jonylidenäthyl-triphenylphosphoniumhydrogensulfat kristallisierte teilweise aus und wurde durch Zugabe von 200 ml Wasser in Lösung gebracht.

b) Die gemäß 7a erhaltene Lösung wurde analog Beispiel 1 in der dort beschriebenen Kolonne mit Wasserdampf behandelt. Man erhielt 1 450 g einer wäßrigen Suspension (bzw. Emulsion), die 470 g β-Jonyliden-äthyl-triphenylphosphoniumhydrogensulfat enthielt. Dies entspricht einer Ausbeute von 90%, bezogen auf eingesetztes Vinyl-β-jonol.

## Beispiel 8

a) 700 ml Isobutanol wurden mit 258,5 g Triphenylphosphin versetzt und zu dieser Mischung wie in Beispiel 6 zunächst 99,5 g konzentrierte Schwefelsäure und dann 220 g Vinyl-β-jonol (Reinheitsgrad 93 %) getropft. Anschließend wird das Reaktionsgemisch mit 200 ml Wasser versetzt und für 2 Stunden auf 50°C erwärmt.

b) Die gemäß 8a erhaltene Lösung wurde analog Beispiel 1 in der dort beschriebene Kolonne mit Wasserdampf behandelt. Man erhält 1 500 g einer wäßrigen Suspension (bzw. Emulsion), die 480 g β-Jonyliden-äthyl-triphenylphosphoniumhydrogensulfat enthält. Dies enspricht einer Ausbeute von 90%, bezogen auf eingesetztes Vinyl-β-jonol.

## Beispiel 9

a) 700 ml Aceton wurden mit 258,5 g Triphenylphosphin versetzt und zu dieser Mischung wie in Beispiel 6 zunächst 99,5 g konzentrierte Schwefelsäure und dann 220 g Vinyl-β-jonol zugetropft. Anschließend wurde das Reaktionsgemisch mit 100 ml Wasser versetzt, für 2 Stunden auf 30°C erwärmt und noch 20 Stunden nachreagieren lassen.

b) Die gemäß 9a erhaltene Lösung wurde analog Beispiel 1 in der dort beschriebenen Kolonne mit Wasserdampf behandelt. Man erhält 1 450 g einer wäßrigen Suspension (bzw. Emulsion), die 470 g β-Jonylidenäthyl-triphenylphosphonium-hydrogensulfat enthält. Das entspricht einer Ausbeute von 90%, bezogen auf eingesetztes Vinyl-β-jonol.

## Beispiel 10

a) 700 ml Methanol wurden mit 50 ml Pyridin, 50 ml konzentrierter wäßriger HCl und 6 ml einer 10 gewichtsprozentigen Lösung von butyliertem Hydroxyanisol in Benzol versetzt, die Mischung 5 Minuten gerührt und danach gemäß DT—OS 25 37 072 mit139 g Triphenylphosphin und 120 g Vinyl-β-jonol (Reinheitsgrad 93 %) versetzt. Anschließend wurde das Reaktionsgemisch 4 x mit 250 ml Heptan extrahiert.

b) Die gemäß 10a erhaltene methanolische Lösung wurde analog Beispiel 1 in der dort beschriebenen Apparatur mit Wasserdampf behandelt. Man erhält 1 450 g einer wäßrigen Suspension,

die 836 g $\beta$-Jonyliden-äthyl-triphenylphosphoniumchlorid enthält. Das entspricht einer Ausbeute von 83 %, bezogen auf eingesetztes Vinyl-$\beta$-jonol.

Beispiel 11

800 ml Methylenchlorid wurden mit 262 g Triphenylphosphin versetzt. Zur dieser Lösung wurden unter Rühren und Kühlen 99,5 g konzentrierte Schwefelsäure und danach 220 g Vinyl-$\beta$-jonol bei 10—15°C zugetropft. Man läßt 12 Stunden bei Raumtemperatur nachreagieren. Diese Lösung wurde wie in Beispiel 1 kontinuierlich in 90 Minuten auf die dort beschriebene kolonne aufgegeben.

Gleichzeitig wurden ca. 2 kg Wasserdampf eingeblasen. Man erheielt als Destillat 800 ml Methylenchlorid und ca. 1,3 kg Wasser.

Das Methylenchlorid kann nach Abrennen des Wassers und Destillation wieder eingesetzt werden.

Der Sumpf wurde in einen Rührkolben ablaufen lassen und unter fortwährendem Rühren auf Raumtemperatur abgekühlt. Man erhielt 1 200 g einer gut rührbaren wäßrigen Kristallmaische, die 497 g $\beta$-Jonylidentriphenylphosphoniumhydrogensulfat enthielt.

Dies entspricht einer Ausbeute von 93 % der Theorie.

**Patentansprüche**

1. Verfahren zur Uberführung 10- bis 70 gewichtsprozentiger Lösungen von Polyenyltriarylphosphoniumsalzen der allgemeinen Formel I

$$[R—P(Ar)_3]^+ X^- \qquad (I),$$

in der R für einen aliphatischen, cycloaliphatisch-aliphatischen oder aromatisch-aliphatischen Polyenylrest mit 5 bis 20 C-Atomen steht, X das Anionäquivalent einer starken Säure bezeichnet und Ar einen Arylrest bedeutet, in einem organischen Lösungsmittel in weitgehend lösungsmittelfreie wäßrige Lösungen bzw. feinteilige Dispersionen, *dadurch gekennzeichnet,* daß man das Lösungsmittel sowie sonstige von der Synthese von I herstammenden wasserdampfflüchtigen Verbindungen aus der auf 30 bis 120°C gehaltenen Lösung mit Wasserdampf abtreibt, wobei man einen Teil des Wasserdampfes zur Erzeugung der wäßrigen Lösungen bzw. der feinteiligen Dispersionen kondensieren läßt.

2. Verfahren nach Anspruch 1, *dadurch gekennzeichnet,* daß man die organische Lösung von I von oben kontinuierlich in eine Kolonne leitet, im Gegenstrom kontinuierlich mit dem Wasserdampf in Kontakt bringt, hierbei einen Teil des Wasserdampfes kondensieren läßt und die gebildete wäßrige Lösung von I kontinuierlich aus dem unteren Teil der Kolonne abzieht.

3. Verfahren nach den Ansprüchen 1 und 2, *dadurch gekennzeichnet,* daß man von methanolischen I-Lösung ausgeht.

**Claims**

1. A process for converting 10 to 70 wt. % solutions of polyenyltriarylphosphonium salts of the general formula I

$$[R—P(Ar)_3]^+ X^- \qquad (I),$$

where R denotes an aliphatic, cycloaliphatic-aliphatic or aromatic-aliphatic polyenyl radical of 5 to 20 carbon atoms, X denotes the anion equivalent of a strong acid, and Ar denotes an aryl radical, in an organic solvent into substantially solvent-free aqueous solutions or small particle size dispersions, *characterized in that* the solvent and other compounds volatile with steam and originating from the synthesis of I are expelled with steam from the solution maintained at 30 to 120°C, part of the steam being allowed to condense for the preparation of the aqueous solutions or small particle size dispersion.

2. A process according to claim 1, *characterized in that* the organic solution of I is fed continuously to the top of a column, contacted continuously with the steam in countercurrent, allowing part of the steam to condense, and the resultant aqueous solution of I is continuously withdrawn at the bottom of the column.

3. A process according to claims 1 and 2, *characterized in that* a methanolic solution of I is used as feedstock.

**Revendications**

1. Procédé pour la transformation en solutions aqueuses ou en dispersions aqueuses finement divisées avec une teneur résiduelle limitée en solvant organique de solutions d'environ 10 à 70 % en poids dans un solvant organique de sels de polyényl-triaryl-phosphonium de la formule générale

$$[R—P(Ar)_3]^+ X^- \qquad (I),$$

dans laquelle le symbole R désigne un groupe polyényle aliphatique, cycloaliphatique-aliphatique ou aromatique-aliphatique en $C_5$ à $C_{20}$, le symbole X l'équivalent anionique d'un acide fort et le symbole Ar un groupe aryle, caractérisé en ce que le solvant organique, ainsi que les produits entraînables à la vapeur d'eau, formés lors de la synthèse du composé de la formule I, sont entrainés à la vapeur d'eau de la solution maintenue entre 30 et 120°C, une fraction de la vapeur étant condensée pour l'obtention de la solution aqueuse ou dispersion aqueuse finement divisée du composé de la formule I.

2. Procédé suivant la revendication 1, caractérisé en ce que la solution organique d'un composé de la formule I est introduite en continu en tête d'une colonne, traversée en continu d'un contre-courant de vapeur d'eau, dont une fraction est condensée, la solution aqueuse du composé de la formule I étant soutirée en continu à la base de la colonne.

3. Procédé suivant l'une quelconque des revendications 1 et 2, caractérisé en ce que la solution organique de départ du composé de la formule I est une solution méthanolique.